(19) Europäisches Patentamt
European Patent Office
Office européen des brevets

(11) EP 2 524 911 A1

(12) **EUROPEAN PATENT APPLICATION**
published in accordance with Art. 153(4) EPC

(43) Date of publication:
**21.11.2012 Bulletin 2012/47**

(21) Application number: **11731856.8**

(22) Date of filing: **07.01.2011**

(51) Int Cl.:
***C07D 207/10*** *(2006.01)*

(86) International application number:
**PCT/JP2011/050206**

(87) International publication number:
**WO 2011/083858 (14.07.2011 Gazette 2011/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.01.2010 JP 2010002665**

(71) Applicant: **Kyorin Pharmaceutical Co., Ltd.**
**Tokyo 101-8311 (JP)**

(72) Inventors:
- **ARAYA Ichiro**
  **Tochigi 329-0114 (JP)**
- **ORITA Kazuo**
  **Saitama 349-0141 (JP)**

(74) Representative: **Vossius & Partner**
**Siebertstrasse 4**
**81675 München (DE)**

# (54) METHOD FOR PRODUCING 3,4-DISUBSTITUTED PYRROLIDINE DERIVATIVE AND PRODUCTION INTERMEDIATE THEREOF

(57) Provided is an inexpensive and industrially advantageous method for preparing (3R,4S)-3-(N-substituted aminomethyl)-4-fluoropyrrolidine or an enantiomer thereof which can be an intermediate for producing pharmaceuticals. It relates to a method for preparing (3R,4S)-3-(N-substituted cyclopropylaminomethyl)-4-fluoropyrrolidine derivative or ait's enantiomer, or their salts, comprising a step of fluorinating a 4-hydroxy-3-(N-substituted aminomethyl)pyrrolidine derivative represented by the general formula (1) or an enantiomer thereof using a sulfur tetrafluoride derivative.

[Chem. 1]

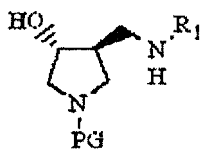

(1)

[In the formula (1), PG represents a protective group for the amino group, and $R_1$ represents a C1 to C6 alkyl group which may be substituted, or a C3 to C8 cycloalkyl group which may be substituted.]

EP 2 524 911 A1

## Description

### Technical Field

The present invention relates to a novel method for preparing an optically-active form of 3-(N-substituted aminomethyl)-4-fluoropyrrolidine, which is an intermediate useful for preparing a 7-(3-(N-substituted aminomethyl)-4-fluoropyrrolidinyl)quinolone carboxylic acid derivative, which is safe, shows potent antibacterial activity, and also is effective to resistant bacteria on which conventional antibacterial agents hardly show their effects, as well as an intermediate for preparing the same.

### Background Art

Patent Documents 1 and 2 disclose a 10-(3-cyclopropylaminomethyl-4-substituted 1-pyrrolidinyl)pyridobenzoxazinecarboxylic acid derivative and a 7-(3-cyclopropylaminomethyl-4-substituted 1-pyrrolidinyl)quinolonecarboxylic acid derivative, as antimicrobial agents which show excellent antibacterial activity on resistant bacteria and have high safety.

These Patent Documents disclose a method for preparing a useful intermediate, (3R,4S)-3-cyclopropylaminomethyl-4-fluoropyrrolidine, wherein the synthesis pathway of the intermediate requires nine steps, starting from N-methoxymethyl-N-(trimethylsilylmethyl)benzylamine as a starting material, including fluorination of 3-azidomethyl-4-hydroxypyrrolidine, and reaching synthesis of (3R,4S)-3-cyclopropylaminomethyl-4-fluoropyrrolidine.

Patent Documents 3 and 4 also disclose improved methods for preparation of (3R,4S)-3-cyclopropylaminomethyl-4-fluoropyrrolidine. These modified methods conduct short steps compared to the conventional methods. However, in Patent Document 3, all the intermediates are oily products, and are difficult to purify. In Patent Document 4, since it is necessary to protect an amino group before the fluorination reaction, an extremely large amount of time is required to perform protection and deprotection steps, which leads to a reduction in yield.

Meanwhile, the prior documents and prior art regarding the method for preparing fluoroamine from amino alcohol includes a synthesis method of once protecting an amino group followed by fluorination and deprotection of the amino group (Patent Documents 5 and 6, Non-Patent Documents 1 and 2), but is not desirable due to an increase in the number of steps.

As a prior art of directly fluorinating an amino alcohol with an amino group being unprotected, a method using a hydrogen fluoride pyridine complex (Non-Patent Document 3), a method using a sulfur tetrafluoride derivative (Patent Document 7), and a method using sulfur tetrafluoride and liquid hydrogen fluoride (Non-Patent Documents 4 and 5) have been reported.

The method of Non-Patent Document 3 has a problem in that, although the yield is relatively high, it takes several days to complete the reaction. Further, in Patent Document 7, a sulfur tetrafluoride derivative, (diethylamino) sulfur trifluoride (DAST), is used to convert a benzyl 2-hydroxyethylamine derivative into a benzyl 2-fluoroethylamine derivative, but the yield is as low as 17%.

Non-Patent Documents 4 and 5 disclose a method for obtaining amine fluoride by reacting sulfur tetrafluoride in liquid hydrogen fluoride at -78°C. However, since 4-fluoropiperidine is produced from 3-hydroxypiperidine through rearrangement at the fluorination position, the optical purity of the product is decreased when an optically active starting material is used.

### Citation List

#### Patent Document

Patent Document 1: WO03/07843
Patent Document 2: WO05/026147
Patent Document 3: JP-A-2005-239617
Patent Document 4: WO07/102567
Patent Document 5: WO05/075426
Patent Document 6: JP-A-2008-222578
Patent Document 7: WO06/13048

#### Non-Patent Document

Non-Patent Document 1: Journal of Labelled Compounds & Radiopharmaceuticals, (2000), 43(10), 1047-1058.
Non-Patent Document 2: Tetrahedron Letters, (1996), 37(43), 7743-7744.
Non-Patent Document 3: J. Chem. Res., 246, 1983.
Non-Patent Document 4: Journal of Organic Chemistry, (1979), 44(5), 771-777.
Non-Patent Document 5: Journal of Organic Chemistry, (1975), 44(25), 3808-3809.

Summary of Invention

Problems to be Solved by the Invention

An inexpensive and industrially advantageous method for preparing high-quality (3R,4S)-3-(N-substituted aminomethyl)-4-fluoropyrrolidine or it's enantiomer, or their salts, which are useful as intermediates for producing pharmaceuticals is provided.

Means for Solving the Problems

To solve the above problems, the present inventors have conducted extensive researches on processes of a novel synthesis method for (3R,4S)-3-(N-substituted aminomethyl)-4-fluoropyrrolidine or an enantiomer thereof.
As a result, a highly pure (3S,4S)-3-(N-substituted aminomethyl)-4-fluoropyrrolidine derivative was successfully obtained at a high yield by fluorinating (3S,4R)-4-hydroxy-3-(N-substituted aminomethyl)pyrrolidine, which has a protective group on the 1-position, using a sulfur tetrafluoride derivative.
Further, by removing the protective group at the 1-position of the obtained (3S,4S)-3-(N-substituted aminomethyl)-4-fluoropyrrolidine derivative, an inexpensive and industrially advantageous method for preparing (3R,4S)-3-(N-substituted aminomethyl)-4-fluoropyrrolidine was found, and therefore the present invention was completed.
In addition, the reaction proceeds in the same manner using (3R,4S)-4-hydroxy-3-(N-substituted aminomethyl) pyrrolidine having a protective group at the 1-position, which is an enantiomer of (3S,4R)-4-hydroxy-3-(N-substituted aminomethyl)pyrrolidine having a protective group at the 1-position.
That is, the present invention includes the following inventions.

1. A method for preparing a compound represented by the following general formula (2) or it's enantiomer, or their salts, comprising:

   a fluorination step of fluorinating a 4-hydroxy-3-(N-substituted aminomethyl)pyrrolidine derivative represented by the following general formula (1) or an enantiomer thereof using a sulfur tetrafluoride derivative

[Chem. 1]

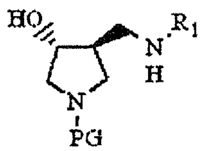

(1)

[In the formula (1), PG represents a protective group for the amino group, and $R_1$ represents a C1 to C6 alkyl group which may be substituted, or a C3 to C8 cycloalkyl group which may be substituted.]
[Chem. 2]

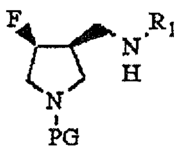

(2)

[In the formula (2), PG represents a protective group for the amino group, and $R_1$ represents a C1 to C6 alkyl group which may be substituted, or a C3 to C8 cycloalkyl group which may be substituted.].

2. The production method describrd in 1 above, further comprising, after the fluorination step:

adding an acid to obtain a salt of a compound represented by the general formula (2) or a salt of an enantiomer thereof, and
further purifying the salt,
wherein the salt of the compound represented by the general formula (2) or the salt of the enantiomer is produced at an HPLC relative purity of 98% or more.

3. A method for preparing a compound represented by the following general formula (3) or it's enantiomer, or their salts, comprising step 1 and step 2:

(Step 1) a fluorination step of obtaining a compound represented by the following general formula (2) or it's enantiomer, or their salts by fluorinating the derivative of 4-hydroxy-3-(N-substituted aminomethyl)pyrrolidine represented by the following general formula (1) or an enantiomer thereof using a sulfur tetrafluoride derivative; and
(Step 2) a deprotection step of obtaining a compound represented by the following general formula (3) or it's enantiomer, or their salts by deprotecting the protective group on the amino group of the compound represented by the following general formula (2) or it's enantiomer, or their salts obtained in step 1.

[Chem. 3]

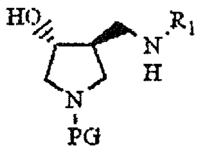
(1)

[In the formula (1), PG represents a protective group for the amino group, and $R_1$ represents a C1 to C6 alkyl group which may be substituted, or a C3 to C8 cycloalkyl group which may be substituted.]
[Chem. 4]

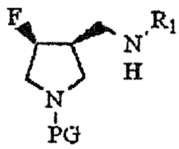
(2)

[In the formula (2), PG represents a protective group for the amino group, and $R_1$ represents a C1 to C6 alkyl group which may be substituted, or a C3 to C8 cycloalkyl group which may be substituted.]
[Chem. 5]

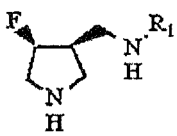
(3)

[In the formula (3), $R_1$ represents a C1 to C6 alkyl group which may be substituted, or a C3 to C8 cycloalkyl group which may be substituted.]

4. The production method described in any one of 1 to 3 above, wherein water or a hydrogen fluoride source is added in the fluorination step.
5. The production method described in any one of 1 to 4 above, wherein a sulfur tetrafluoride derivative is added in the fluorination step after the addition of the water or hydrogen fluoride source.
6. The production method described in 4 or 5 above, wherein the hydrogen fluoride source is a hydrogen fluoride pyridine complex or a triethylamine pentahydrofluoride.
7. The production method described in any one of 4 to 6 above, wherein the hydrogen fluoride source is the triethylamine pentahydrofluoride.
8. The production method described in any one of 1 to 7 above, wherein the hydrogen fluoride source is used at 3 equivalents or more with respect to the 4-hydroxy-3-(N-substituted aminomethyl)pyrrolidine derivative represented by the general formula (1) or the enantiomer thereof in the fluorination step.
9. The production method described in any one of 1 to 8 above, wherein the hydrogen fluoride source is used at 4 equivalents or more with respect to the 4-hydroxy-3-(N-substituted aminomethyl)pyrrolidine derivative represented by the general formula (1) or the enantiomer thereof in the fluorination step.
10. The production method described in any one of 1 to 9 above, wherein the sulfur tetrafluoride derivative is bis(2-methoxyethyl)aminosulfur trifluoride (Deoxo-Fluor).
11. The production method described in any one of 1 to 10 above, wherein the protective group for the amino group represented by PG is an alkoxycarbonyl group or an aralkoxycarbonyl group.
12. The production method described in 11 above, wherein the protective group for the amino group represented by PG is a benzyloxycarbonyl group.
13. The production method described in any one of 1 to 12 above, wherein $R_1$ is a cyclopropyl group.
14. The production method described in any one of 1 to 10 above, wherein the protective group for the amino group represented by PG is a benzyloxycarbonyl group, and $R_1$ is a cyclopropyl group.
15. A hydrochloride of a compound represented by the general formula (2) or a hydrochloride of an enantiomer thereof

[Chem. 6]

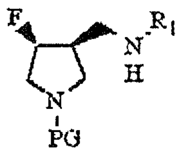

(2)

[In the formula (2), PG represents a protective group for the amino group, and $R_1$ represents a C1 to C6 alkyl group which may be substituted, or a C3 to C8 cycloalkyl group which may be substituted.].

16. A method for purifying a hydrochloride of a compound represented by the general formula (2) or a hydrochloride of an enantiomer thereof to increase the purity

[Chem. 7]

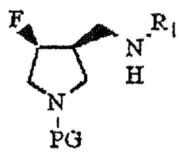

(2)

[In the formula (2), PG represents a protective group for an amino group, and $R_1$ represents a C1 to C6 alkyl group which may be substituted, or a C3 to C8 cycloalkyl group which may be substituted.].

Effects of the Invention

According to the present invention, a compound represented by the general formula (3) or an enantiomer thereof can be effectively produced under a moderate condition. As a result, a large amount of the compound represented by the general formula (3) or an enantiomer thereof can be produced inexpensively.

Embodiments for Carrying Out the Invention

Hereinafter, the present invention will be described in detail.

The present invention provides a method for preparing a compound represented by the following general formula (2) or it's enantiomer, or their salts, comprising a fluorination step of fluorinating a 4-hydroxy-3-(N-substituted aminomethyl)pyrrolidine derivative represented by the following general formula (1) or an enantiomer thereof using a sulfur tetrafluoride derivative.

[Chem. 8]

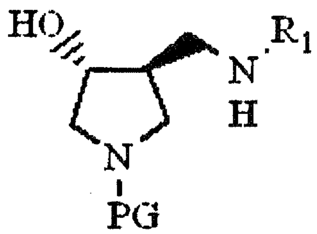

(1)

In the formula (1), PG represents a protective group for the amino group, and $R_1$ represents a C1 to C6 alkyl group which may be substituted, or a C3 to C8 cycloalkyl group which may be substituted. In the compound represented by the general formula (1), it is preferred that the amino group binding to $R_1$ is not protected, and is a compound other than a salt such as hydrochloride.

[Chem. 9]

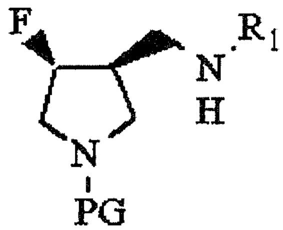

(2)

In the general formula (2), PG represents a protective group for the amino group, and $R_1$ represents a C1 to C6 alkyl group which may be substituted, or a C3 to C8 cycloalkyl group which may be substituted.

Further, the present invention provides a method for preparing a compound represented by the following general formula (3) or it's enantiomer, or their salts, including step 1 and step 2.

(Step 1) A fluorination step of obtaining a compound represented by the general formula (2) or it's enantiomer, or their salts, by fluorinating the 4-hydroxy-3-(N-substituted aminomethyl)pyrrolidine derivative represented by the general formula (1) or an enantiomer thereof using a sulfur tetrafluoride derivative.

(Step 2) A deprotection step of obtaining a compound represented by the following general formula (3) or an enantiomer thereof, or salts thereof, by deprotection of the protective group on the amino group of the compound represented by the general formula (2) or it's enantiomer, or their salts obtained in step 1.

[Chem. 10]

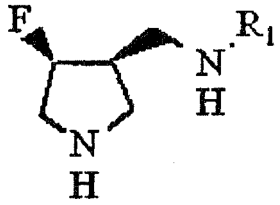

(3)

In the formula (3), $R_1$ represents a C1 to C6 alkyl group which may be substituted, or a C3 to C8 cycloalkyl group which may be substituted.

In the formula (1) and the formula (2), the "protective group for the amino group" is not particularly limited so long as the protective group is generally known as a protective group for the amino group, and examples thereof include an aralkyl group such as a benzyl group and a paramethoxybenzyl group, an alkoxycarbonyl group such as a methoxycarbonyl group, an ethoxycarbonyl group, a propyloxycarbonyl group, an isopropyloxycarbonyl group, a butyloxycarbonyl group, an isobutyloxycarbonyl group and a t-butyloxycarbonyl group, an aralkoxycarbonyl group such as a benzyloxycarbonyl group, a p-methoxybenzyloxycarbonyl group and a p-nitrobenzyloxycarbonyl group, a 1-(alkoxy)alkyl group such as a methoxymethyl group, a methoxyethoxymethyl group, a 1-(ethoxy)ethyl group and a methoxyisopropyl group, and an acyl group such as an acetyl group, a trifluoroacetyl group, a propionyl group, a butyryl group, a pivaloyl group, a benzoyl group and a methylbenzoyl group.

Among these, an aralkoxycarbonyl group or an alkoxycarbonyl group is particularly preferred, and an aralkoxycarbonyl group is more preferred. Examples of the particularly preferred protective group include a benzyloxycarbonyl group.

In the formula (1) to the formula (3), the "C1 to C6 alkyl group which may be substituted" means a C1 to C6 alkyl group which may have 1 to 5 substituents selected from the group consisting of a halogen atom, a hydroxyl group, a cyano group, a C1 to C6 alkoxy group, an aryloxy group which may be substituted, a C1 to C6 alkylcarbonyl group, a C1 to C6 alkoxycarbonyl group, a C1 to C6 alkylthio group, an amino group, a mono- or di-substituted C1 to C6 alkylamino group, a C4 to C9 cyclic amino group which may have 1 to 3 heteroatoms, a formylamino group, a C1 to C6 alkylcarbonylamino group, a C1 to C6 alkoxycarbonylamino group, a C1 to C6 alkylsulfonylamino group and an arylsulfonylamino group which may be substituted.

The "C1 to C6 alkyl group" means a linear or branched lower alkyl group. Examples of the C1 to C6 alkyl group include a methyl group, an ethyl group, a propyl group, a 1-methylethyl group, a 1-methylpropyl group, a 2-methylpropyl group, a 1-ethylpropyl group, a 2-ethylpropyl group, a butyl group and a hexyl group. Among these, an ethyl group is preferred.

Examples of the "C1 to C6 alkoxy group" include a methoxy group, an ethoxy group, a butoxy group and a hexyloxy group.

The "aryloxy group which may be substituted" means an aryloxy group which may have 1 to 5 substituents selected from the group consisting of a halogen atom, a hydroxyl group, a cyano group, a nitro group, a C1 to C6 alkyl group, a C1 to C6 alkoxy group and a C1 to C6 alkylthio group.

Examples of the "aryloxy group" include a phenoxy group and a naphthyloxy group.

Examples of the "C1 to C6 alkylcarbonyl group" include a formyl group, an acetyl group, a propionyl group, a butyryl group, an isobutyryl group, a valeryl group and an isovaleryl group.

Examples of the "C1 to C6 alkoxycarbonyl group" include a methoxycarbonyl group, an ethoxycarbonyl group and a tert-butoxycarbonyl group.

Examples of the "C1 to C6 alkylthio group" include a methylthio group, an ethylthio group, a propylthio group and an isopropylthio group.

The "mono- or di-substituted C1 to C6 alkylamino group" means a C1 to C6 alkylamino group which may have 1 to 2 substituents selected from the group consisting of a halogen atom, a hydroxyl group, a cyano group, a C1 to C6 alkoxy group, a C1 to C6 alkylthio group, an amino group, a C4 to C9 cyclic amino group which may have 1 to 3 heteroatoms, a formylamino group, a C1 to C6 alkylcarbonylamino group, a C1 to C6 alkylsulfonylamino group and an arylsulfonylamino group which may be substituted.

Examples of the "C1 to C6 alkylamino group" include a methylamino group, an ethylamino group, an n-propylamino group, an n-butylamino group, a sec-butylamino group, an n-pentylamino group and an n-hexylamino group.

The "C4 to C9 cyclic amino group" means a cyclic amino group which contains at least one nitrogen atom in the ring and in which an oxygen atom and a sulfur atom may also be present in the ring. Examples of the C4 to C9 cyclic amino group include an aziridyl group, a pyrrolidyl group, a piperidyl group, a morpholyl group, an oxazolyl group, an

azabicycloheptyl group and an azabicyclooctyl group.

Examples of the "C1 to C6 alkylcarbonylamino group" include an acetylamino group, a propionylamino group and a butyrylamino group.

Examples of the "C1 to C6 alkoxycarbonylamino group" include a methoxycarbonylamino group, an ethoxycarbonylamino group, a t-butoxycarbonylamino group and a hexyloxycarbonylamino group.

Examples of the "C1 to C6 alkylsulfonylamino group" include a methylsulfonylamino group, an ethylsulfonylamino group, etc.

The "arylsulfonylamino group which may be substituted" means an arylsulfonylamino group which may have 1 to 5 substituents selected from the group consisting of, for example, a halogen atom, a hydroxyl group, a cyano group, a nitro group, a C1 to C6 alkyl group, a C1 to C6 alkoxy group and a C1 to C6 alkylthio group.

Examples of the "arylsulfonylamino group" include a phenylsulfonylamino group, a 4-methylphenylsulfonylamino group and a naphthylsulfonylamino group.

Examples of the "C3 to C8 cycloalkyl group which may be substituted" include a C3 to C8 cycloalkyl group which may have 1 to 5 substituents selected from the group consisting of a halogen atom, a hydroxyl group, a cyano group, a C 1 to C6 alkoxy group, an aryloxy group which may be substituted, a C1 to C6 alkylcarbonyl group, a C 1 to C6 alkoxycarbonyl group, a C 1 to C6 alkylthio group, an amino group, a mono- or di-substituted C1 to C6 alkylamino group, a C4 to C9 cyclic amino group which may have 1 to 3 heteroatoms, a formylamino group, a C 1 to C6 alkylcarbonylamino group, a C 1 to C6 alkoxycarbonylamino group, a C 1 to C6 alkylsulfonylamino group and an arylsulfonylamino group which may be substituted.

The "C3 to C8 cycloalkyl group" means an alkyl group having a cycloalkyl ring. Examples of the C3 to C8 cycloalkyl group include a cyclopropyl group, a cyclopropylmethyl group, a cyclobutyl group, a cyclopentyl group, a cyclohexyl group, a cycloheptyl group and a cyclooctyl group.

The preparation method of the present invention is shown in Scheme 1.

[Chem. 11]

Scheme 1

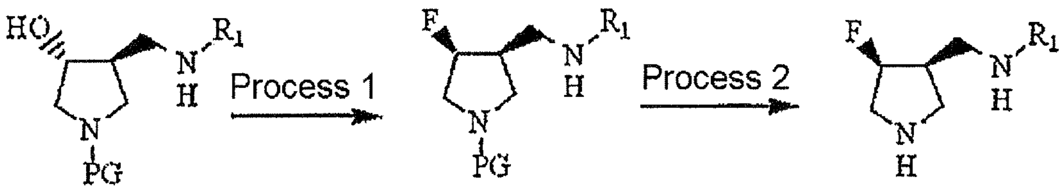


(1) (2) (3)

In the scheme 1, PG in the formula (1) and the formula (2) and $R_1$ in the formula (1) to the formula (3) have the same meanings as described above.

I. Step 1

Step 1 is a step of changing a hydroxyl group of the compound represented by the general formula (1) or an enantiomer thereof into a fluorine group by fluorinating the 4-hydroxy-3-(N-substituted aminomethyl)pyrrolidine derivative represented by the general formula (1) or an enantiomer thereof using a sulfur tetrafluoride derivative.

In step 1, the sulfur tetrafluoride derivative functions as a fluorinating agent. Examples of the "sulfur tetrafluoride derivative" include (dimethylamino)sulfur trifluoride (Methyl DAST), (diethylamino)sulfur trifluoride (DAST), morpholinosulfur trifluoride (Morpho-DAST) and bis(2-methoxyethyl)aminosulfur trifluoride (Deoxo-Fluor).

Among these, morpholinosulfur trifluoride (Morpho-DAST) or bis(2-methoxyethyl)aminosulfur trifluoride (Deoxo-Fluor) is preferred.

Generally, the sulfur tetrafluoride derivative is used preferably at an equimolar amount of 1.0 to 10 times with respect to the compound represented by the general formula (1).

In the fluorination step, an amount of the fluorinating agent used may be reduced by adding a hydrogen fluoride source or water. Therefore, the amount of the fluorinating agent used may be reduced to 1.0 to 1.5 equivalents per mole, with respect to the compound represented by the general formula (1), so that the cost can be reduced.

In the fluorination step, the fluorination reaction is accelerated due to the presence of the hydrogen fluoride source or water, and thus 75% or more of the reaction, or 90% or more under more preferred conditions, is completed within 24 hours. The effect to accelerate the reaction is increased by adding a sulfur tetrafluoride derivative after addition of the water or hydrogen fluoride source.

When an excessive amount of the sulfur tetrafluoride derivative is used for the fluorination step, water may be added to a reaction system, or solvent containing water may be used. Existence of water accelerates the reaction.

The "water" includes water already contained in the reaction solvent, as well as water added to a reaction solvent afterward. An amount of water added or a moisture content of a solvent is preferably in a range of 1,000 to 8,000 ppm.

The "hydrogen fluoride source" means hydrogen fluoride, or a salt or a complex made of an organic base and a hydrogen fluoride, which generates free hydrogen fluoride. Examples of the hydrogen fluoride source include a triethylamine hydrofluorides, a hydrogen fluoride pyridine complex and a hydrogen fluoride-melamine complex.

Among these hydrogen fluoride sources, a triethylamine hydrofluorides containing free hydrogen fluoride is preferred, in an aspect that it is possible to accelerate the reaction so as to reduce an amount of the sulfur tetrafluoride derivative, and a triethylamine pentahydrofluoride is particularly preferred. Meanwhile, triethylamine trihydrofluoride does not contain an excessive amount of hydrogen fluoride with respect to triethylamine, and thus, does not show an effect of accelerating the reaction.

Generally, the amount of the hydrogen fluoride source used is preferably in a equivalent range of 1 to 10 times per mole, based on the compound represented by the general formula (1), and more preferably in a range of 3 equivalents or more and further preferably in a range of 4 equivalents or more in an aspect of accelerating the reaction.

For the fluorination step, a reaction solvent may or may not be used. When the reaction solvent is used, a reaction solvent is not particularly limited, so long as it is stable under the reaction conditions and also is inert and does not interfere the reaction.

Examples of the reaction solvent include a solvent such as acetonitrile, toluene, tetrahydrofuran, chloroform, methylene chloride and ethyl acetate. Among these, toluene and acetonitrile are preferred.

The reaction solvent is properly selected depending on the ease of initiating the reaction, and used either alone or in combination. Further, a proper dehydrating agent or desiccant is used as a non-aqueous solvent, as necessary. The amount of the reaction solvent used is not particularly limited, but is preferably in a range of 2.5 to 20 times the weight of the compound represented by the general formula (1) or salts thereof.

A reaction temperature is preferably within a range from -78°C to a boiling point of the solvent used, and more preferably in a range of -10 to 30°C. Meanwhile, the fluorination reaction in the fluorination step does not necessarily require a low-temperature condition such as -78°C because the liquid hydrogen fluoride is not used.

After the fluorination reaction is completed, conversion into a salt of the compound represented by the general formula (2) or a salt of an enantiomer thereof can be effected by adding an acid. Examples of the acid which may be used includes hydrogen chloride, hydrochloric acid, hydrobromic acid, hydroiodic acid, trifluoroacetic acid, trifluoromethanesulfonic acid, methanesulfonic acid and toluenesulfonic acid. Among these, hydrogen chloride or hydrochloric acid is preferred.

The obtained salt may be purified and isolated by using conventional separation methods(for example, extraction, recrystallization and chromatography). A salt of the compound represented by the general formula (2) or a salt of an enantiomer thereof may be obtained at an HPLC relative purity of 98% or more under more preferred conditions.

Examples of the solvent which may be used for purification by recrystallization include water, methanol, ethanol, ethyl acetate, diisopropyl ether, 2-propanol, acetonitrile, chloroform, N,N-dimethyl formamide, dimethyl sulfoxide, N-methyl pyrrolidone, methyl ethyl ketone, tetrahydrofuran and tetrahydropyran.

Among these, alcohols, ether and ethyl acetate are preferred, and methanol, ethanol, diisopropyl ether and ethyl acetate are more preferred. These solvents may be used either alone or in combination.

II. Step 2

Step 2 is a deprotection step of obtaining the compound represented by the general formula (3) or it's enantiomer, or their salts by removing a protective group (PG) on the amino group of the compound represented by the general formula (2) or it's enantiomer, or their salts, obtained in step 1.

For step 2, the removal of the protective group on the amino group of the compound represented by the general formula (2) or it's enantiomer, or their salts may be performed by properly employing a method described in a literature (Green, T. W.; Wuts, P. G. M. "Protective Groups in Organic Synthesis," 2nd Ed., Wiley Interscience Publication, John-Weiley & Sons, New York, 1991, p 309-348.).

II-1. Case in which a protective group is an aralkoxycarbonyl group and an aralkyl group

For example, when the protective group (PG) is an aralkoxycarbonyl group such as a benzyloxycarbonyl group, and an aralkyl group such as a benzyl group and a paramethoxybenzyl group, the protective group (PG) may be removed in the presence of a hydrogen source by catalytic hydrogenation.

Examples of the catalyst used include a palladium catalyst such as palladium on carbon, palladium on alumina, palladium black and palladium oxide. Among these, palladium on carbon is preferred.

Examples of the hydrogen source include hydrogen, a formic acid/triethylamine system, a formic acid/α-phenethylamine system, a formic acid/triphenylamine system and 2-propanol. Among these, hydrogen is preferred.

Generally, hydrogen pressure in the reaction is preferably in a range of atmospheric pressure to 100 atm, more preferably in a range of atmospheric pressure to 10 atm, and further preferably atmospheric pressure.

Generally, the reaction is preferably performed using a solvent. Examples of the solvent include an alcohol such as methanol, ethanol, t-butylalcohol, ethylene glycol and diethylene glycol, water, an ether such as tetrahydrofuran, cyclopentylmethylether, dioxane, dimethoxyethane and diglyme, an aromatic compound such as benzene, toluene and xylene, an ester such as methyl acetate, ethyl acetate, isopropyl acetate and butyl acetate, a hydrocarbon such as hexane, heptane and cyclohexane, and a mixture thereof. Among these, alcohols are preferred, and ethanol is more preferred.

Generally, a reaction temperature is preferably within a range from 0°C to a boiling point of a solvent used, and more preferably within a range from room temperature to a boiling point range of a solvent used.

To prevent decomposition of a product, an acid such as sulfuric acid, hydrochloric acid, phosphoric acid, perchloric acid and trifluoroacetic acid, and a base such as ammonia, pyridine, triethylamine, sodium hydroxide and potassium hydroxide may also be added. Addition of the acid is preferred, and hydrochloric acid is more preferred.

II-2. Case in which a protective group is an aralkoxycarbonyl group and an alkoxycarbonyl group

Further, for example, when the protective group (PG) is an aralkoxycarbonyl group such as a benzyloxycarbonyl group, and an alkoxycarbonyl group such as a t-butoxycarbonyl group, the protective group (PG) may be removed by an organic acid or inorganic acid.

Examples of the acid which may be used include an inorganic acid such as hydrochloric acid, sulfuric acid, hydrogen bromide trifluoromethanesulfonic acid and hydrogen iodide, and an organic acid such as acetic acid, trifluoroacetic acid, trifluoromethanesulfonic acid and p-toluenesulfonic acid. Among these, hydrochloric acid is preferred.

Generally, the reaction is preferably performed using a solvent. Examples of the solvent include water, methanol, ethanol, propanol, butanol, acetonitrile, dioxane, dichloromethane, chloroform, N,N-dimethylformamide, dimethylsulfoxide, N-methylpyrrolidone, sulfolane, methylethylketone, tetrahydropyran, N,N-dimethylformamide, dimethylsulfoxide, N-methylpyrrolidone, sulfolane, methylethylketone, tetrahydropyran and a mixture thereof. Among these, water is preferred.

Generally, a reaction temperature is preferably within a range from 0°C to a boiling point of a solvent used, and more preferably within a range from room temperature to a boiling point of a solvent used.

After the reaction of step 2 is completed, purification and isolation can be carried out using conventional separation methods (for example, extraction, recrystallization, chromatography, etc.), if desired.

Examples of the solvent which may be used, for purification by recrystallization, include water, methanol, ethanol, ethyl acetate, 2-propanol, diisopropyl ether, acetonitrile, chloroform, N,N-dimethylformamide, dimethylsulfoxide, N-methylpyrrolidone, methylethylketone, tetrahydrofuran and tetrahydropyran.

Among these, alcohol, water and ethyl acetate are preferred, and ethanol, water and ethyl acetate are more preferred. These solvents may be used either alone or in combination.

The present invention is a method for preparing (3R,4S)-3-(N-substituted aminomethyl)-4-fluoropyrrolidine or a stereoisomer thereof, or salts thereof. According to the preparation method of the present invention, since an amino group (an amino group binding to $R_1$ in the compound represented by the general formula (1)) may be fluorinated without protection, protection and deprotection steps are not required, and thus, the number of steps is decreased, compared to conventional methods (WO 2003/078439 (Patent Document 1) and WO 2007/102567 (Patent Document 4)).

In the preparation method of the present invention, both the compound represented by the general formula (2) and the compound represented by the general formula (3) may be crystallized and easily purified, compared to the synthesis method disclosed in Japanese Patent Laid-Open No. 2005-239617 (Patent Document 3) in which all the intermediates are oily products.

The prior art of directly fluorinating an amino alcohol with an unprotected amino group includes a method using a hydrogen fluoride pyridine complex (J. Chem. Res., 246, 1983.(Non-Patent Document 3)), a method using a sulfur tetrafluoride derivative (WO 2006/13048 (Patent Document 7)), and a method using sulfur tetrafluoride and liquid hydrogen fluoride (Journal of Organic Chemistry, (1979), 44(5), 771-777 (Non-Patent Document 4) and Journal of Organic Chemistry, (1975), 44(25), 3808-3809 (Non-Patent Document 5)).

The method disclosed in J. Chem. Res., 246, 1983 (Non-Patent Document 3) has problems in that, although the yield in the fluorination reaction is relatively excellent, it takes several days to complete a reaction. In the preparation method of the present invention, the reaction may be accelerated in the presence of water or a hydrogen fluoride reaction source so that the reaction time can be shortened to 24 hours or less. Further, the addition of the water or hydrogen fluoride source may reduce an amount of a fluorinating agent used, thereby saving the costs.
WO 2006/13048 (Patent Document 7) discloses that a sulfur tetrafluoride derivative, DAST, is used to convert a benzyl-(2-hydroxy)-ethylamine derivative into a benzyl 2-fluoroethylamine derivative, but the yield is as low as 17%. On the other hand, the fluorination is performed in good yield in the case of the preparation method of the present invention.
In the methods described in Journal of Organic Chemistry, (1979), 44(5), 771-777 (Non-Patent Document 4) and Journal of Organic Chemistry, (1975), 44(25), 3808-3809 (Non-Patent Document 5), a low-temperature reaction is required, and thus, the optical purity is degraded due to the rearrangement of the fluorination position and the racemization. In the case of the preparation method of the present invention, the low-temperature condition is not required, and the rearrangement or racemization does not occure, which make it possible to obtain a desired steric compound (2) or it's stereoisomer, or their salts

EXAMPLE

The present invention will be described with reference to Examples, but the present invention is not limited to these Examples. Benzyl (3S,4R)-3-(cyclopropylaminomethyl)-4-hydroxypyrrolidine-1-carboxylate hydrochloride or benzyl (3R,4S)-3-(cyclopropylaminomethyl)-4-hydroxypyrrolidine-1-carboxylate hydrochloride, which are used as starting materials in Reference Example and Examples, may also be produced according to a method described in WO 2007/102567 (Patent Document 4).

(Reference Example)

Benzyl (3S,4R)-3-(cyclopropylaminomethyl)-4-hydroxypyrrolidine-1-carboxylate

[Chem. 12]

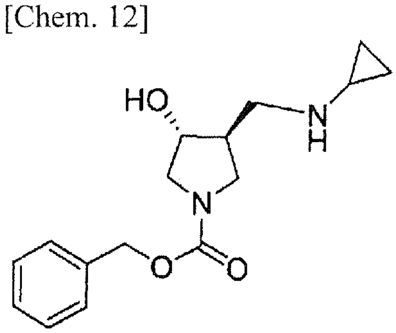

490 g (1.50 mol) of benzyl (3S,4R)-3-(cyclopropylaminomethyl)-4-hydroxypyrrolidine-1-carboxylate hydrochloride was dissolved in 1.47 L of water, 245 g of sodium chloride was added, and 1.13 L of a 2 mol/L sodium hydroxide solution was added at an internal temperature of 2.7 to 10°C under an ice water-cooling condition, with stirring. The mixture was extracted with 2.45 L of ethyl acetate, an organic layer was washed with 2.45 L of saturated saline, and 245 g of anhydrous sodium sulfate was added, followed by stirring for 0.5 hours. Anhydrous sodium sulfate was separated by filtration, a solid was then washed with 0.49 L of ethyl acetate, and the filtrate and the washing were combined, and then concentrated under reduced pressure to obtain 454 g of the title compound as a colorless oily product.
$^1$H-NMR($CDCl_3$, 400MHz) δ: 0.25-0.55(4H, m), 2.06-2.21(1H, m), 2.66(1H, t, J=11.2Hz), 2.96-3.12(2H, m), 3.20(1H, ddd, J=4.6, 7.8, 15.4Hz), 3.65(1H, ddd, J=5.5, 8.4, 16.5Hz), 3.76-3.83(1H, m), 4.07(1H, dd, J=7.3, 14.7Hz), 5.09-5.16(2H, m), 7.28-7.39(5H, m).
HPLC relative purity: 98.2% (RT: 2.74 min) measurement wavelength: 210 nm, column: Inertsil ODS-3, 4.6 mm×150 mm, pre-column: Inertsil ODS-3, 4.0 mm×10 mm, mobile phase: diluted phosphoric acid (1→1000) (solution A), acetonitrile (solution B), 0 to 10 min; linear gradient of solution A:solutin B = from 70:30 to 60:40, 10 to 20 min; linear gradient of solution A:solution B = from 60:40 to 5:95, 20 to 30 min; solution A:solution B=5:95, column temperature: 30°C, flow rate: 1.0 mL/min
Meanwhile, in Examples and Comparative Examples, the "equivalents" of a fluorinating agent and an additive were calculated based on the number of moles of benzyl (3S,4R)-3-(cyclopropylaminomethyl)-4-hydroxypyrrolidine-1-carboxylate on the assumption that benzyl (3S,4R)-3-(cyclopropylaminomethyl)-4-hydroxypyrrolidine-1-carboxylate hydrochloride is converted into benzyl (3S,4R)-3-(cyclopropylaminomethyl)-4-hydroxypyrrolidine-1-carboxylate under the conditions described in Reference Example.

Further, in Examples and Comparative Example, the "multiple" means a volume (mL) of a solvent used with respect to the weight (g) of benzyl (3S,4R)-3-(cyclopropylaminomethyl)-4-hydroxypyrrolidine-1-carboxylate hydrochloride, which is a starting material for benzyl (3S,4R)-3-(cyclopropylaminomethyl)-4-hydroxypyrrolidine-1-carboxylate, and was calculated from the following equation.

Multiple (v/w) = (Volume of solvent) (mL)/(benzyl (3S,4R)-3-(cyclopropylaminomethyl)-4-hydroxypyrrolidine-1-carboxylate hydrochloride used for a reaction) (g)

(Example 1)

Benzyl (3S,4R)-3-(cyclopropylaminomethyl)-4-hydroxypyrrolidine-1-carboxylate obtained by the method described in Reference Example was dissolved in a solvent (acetonitrile, 6 multiples). A fluorinating agent (Deoxo-Fluor, 4 equivalents) was added at an internal temperature of-5 to 5°C, followed by stirring at an internal temperature of -5 to 5°C for 1 hour, and the temperature was raised, followed by stirring at an internal temperature of 10 to 20°C for 7 hours, and then left standing overnight at room temperature (Table 1). The reaction solution was subjected to measurements using HPLC. The results are listed in Table 7.

(Example 2)

Benzyl (3S,4R)-3-(cyclopropylaminomethyl)-4-hydroxypyrrolidine-1-carboxylate obtained by the method described in Reference Example was dissolved in a solvent (acetonitrile. 6 multiples). Thereafter, an additive (triethylamine pentahydrofluoride, 2 equivalents) was added at an internal temperature of -5 to 10°C, followed by stirring at an internal temperature of -5 to 5°C for 0.5 hours. Then, a fluorinating agent (Deoxo-Fluor) was added at an internal temperature of-5 to 5°C, following by stirring at an internal temperature of -5 to 5°C for 1 hour, and the temperature was then raised, followed by stirring at an internal temperature of 10 to 20°C for 7 hours, and then left standing overnight at room temperature (Table 1). The reaction solution was subjected to measurements using HPLC. The results are listed in Table 7.

(Examples 3 to 7)

This reaction was performed in the same manner as in Example 2 using fluorinating agents, additives and solvents listed in Table 1. The reaction solution was subjected to measurements using HPLC. The results are listed in Table 7.

[Table 1]

| | Fluorinating agent (Equivalent) | Additive (Equivalent) | Solvent |
|---|---|---|---|
| Example 1 | Deoxo-Fluor (4.0) | - | $CH_3CN$ |
| Example 2 | Deoxo-Fluor (4.0) | $5HF \cdot Et_3N$ (2.0) | $CH_3CN$ |
| Example 3 | Deoxo-Fluor (1.2) | $5HF \cdot Et_3N$ (10.0) | Toluene |
| Example 4 | DAST (1.2) | $5HF \cdot Et_3N$ (10.0) | Toluene |
| Example 5 | DAST (1.2) | $5HF \cdot Et_3N$ (4.0) | Toluene |
| Example 6 | Morpho-DAST (1.2) | $5HF \cdot Et_3N$ (4.0) | Toluene |
| Example 7 | Deoxo-Fluor (1.2) | $5HF \cdot Et_3N$ (4.0) | Toluene |

(Example 8)

Benzyl (3S,4R)-3-(cyclopropylaminomethyl)-4-hydroxypyrrolidine-1-carboxylate obtained by the method described in Reference Example was dissolved in acetonitrile (6 multiples). A proper amount of purified water was added to the solution (moisture content before addition of purified water: 426 ppm) to adjust a moisture content of the sample solution to 1,194 ppm. Meanwhile, a moisture value was measured using a Karl Fischer method. The reaction was performed on the moisture-containing sample solution in the same manner as in Example 1 (Table 2). The results are listed in Table 8.

(Example 9)

Benzyl (3S,4R)-3-(cyclopropylaminomethyl)-4-hydroxypyrrolidine-1-carboxylate obtained by the method described in Reference Example was dissolved in acetonitrile (6 multiples). A proper amount of purified water was added to the solution (moisture content before addition of purified water: 426 ppm) to adjust a moisture content of the sample solution to 7,358 ppm. Meanwhile, a moisture value was measured using a Karl Fischer method. The reaction was performed on the moisture-containing sample solution in the same manner as in Example 1 (Table 2). The results are listed in Table 8.

(Examples 10 to 11)

This reaction was performed in the same manner as in Example 2 using fluorinating agents, additives and solvents listed in Table 2. The reaction solution was subjected to measurements using HPLC. The results are listed in Table 8.

[Table 2]

| | Fluorinating agent (Equivalent) | Additive (Equivalent) | Solvent |
|---|---|---|---|
| Example 8 | Deoxo-Fluor (4.0) | - | $CH_3CN$ (1,194 ppm, $H_2O$) |
| Example 9 | Deoxo-Fluor (4.0) | - | $CH_3CN$ (7,358 ppm, $H_2O$) |
| Example 10 | Deoxo-Fluor (4.0) | $5HF\cdot Et_3N$ (1.0) | $CH_3CN$ |
| Example 11 | Deoxo-Fluor (1.4) | HF·Pyridine (4.0) | Toluene |

(Examples 12 to 21)

This reaction was performed in the same manner as in Example 2 using fluorinating agents, additives and solvents listed in Table 3. The reaction solution was subjected to measurements using HPLC. The results are listed in Table 9.

[Table 3]

| | Fluorinating agent (Equivalent) | Additive (Equivalent) | Solvent |
|---|---|---|---|
| Example 12 | Deoxo-Fluor (4.0) | $5HF\cdot Et_3N$ (2.0) | $CH_3CN$ |
| Example 13 | Deoxo-Fluor (1.5) | $5HF\cdot Et_3N$ (2.0) | $CH_3CN$ |
| Example 14 | Deoxo-Fluor (1.2) | $5HF\cdot Et_3N$ (2.0) | $CH_3CN$ |
| Example 15 | Deoxo-Fluor (1.0) | $5HF\cdot Et_3N$ (2.0) | $CH_3CN$ |
| Example 16 | Deoxo-Fluor (1.0) | $5HF\cdot Et_3N$ (2.4) | $CH_3CN$ |
| Example 17 | Deoxo-Fluor (1.0) | $5HF\cdot Et_3N$ (3.0) | $CH_3CN$ |
| Example 18 | Deoxo-Fluor (1.2) | $5HF\cdot Et_3N$ (4.0) | $CH_3CN$ |
| Example 19 | Deoxo-Fluor (1.5) | $5HF\cdot Et_3N$ (4.0) | $CH_3CN$ |
| Example 20 | Deoxo-Fluor (1.2) | $5HF\cdot Et_3N$ (3.4) | $CH_3CN$ |
| Example 21 | Deoxo-Fluor (1.2) | $5HF\cdot Et_3N$ (10.0) | Toluene |

(Examples 22 to 25)

This reaction was performed in the same manner as in Example 1 using fluorinating agents and solvents listed in Table 4. The reaction solution was subjected to measurements using HPLC. The results are listed in Table 10.

(Examples 26 to 29, 31)

This reaction was performed in the same manner as in Example 2 using fluorinating agents, additives and solvents listed in Table 4. The reaction solution was subjected to measurements using HPLC. The results are listed in Table 10.

(Example 30)

This reaction was performed in the same manner as in Example 2 using fluorinating agents, additives and solvents listed in Table 4. However, the solvent was used at 12 multiples. The reaction solution was subjected to measurements using HPLC. The results are listed in Table 10.
[Table 4]

| | Fluorinating agent (Equivalent) | Additive (Equivalent) | Solvent |
|---|---|---|---|
| Example 22 | Deoxo-Fluor (4.0) | - | THF |
| Example 23 | Deoxo-Fluor (4.0) | - | DME |
| Example 24 | Deoxo-Fluor (4.0) | - | $CH_2Cl_2$ |
| Example 25 | Deoxo-Fluor (4.0) | - | Toluene |
| Example 26 | Deoxo-Fluor (1.2) | $5HF\cdot Et_3N$ (4.0) | $CHCl_3$ |
| Example 27 | Deoxo-Fluor (1.2) | $5HF\cdot Et_3N$ (4.0) | THF |
| Example 28 | Deoxo-Fluor (1.5) | $5HF\cdot Et_3N$ (4.0) | $CHCl_3$ |
| Example 29 | Deoxo-Fluor (1.5) | $5HF\cdot Et_3N$ (4.0) | Toluene |
| Example 30 | Deoxo-Fluor (1.4) | $5HF\cdot Et_3N$ (4.0) | Toluene |
| Example 31 | Deoxo-Fluor (1.4) | $5HF\cdot Et_3N$ (4.0) | AcOEt |
| Example 32 | Deoxo-Fluor (1.2) | $5HF\cdot Et_3N$ (4.0) | - (neat) |

(Example 32)

Triethylamine pentahydrofluoride (4 equivalents) was added dropwise to (3S,4R)-3-(cyclopropylaminomethyl)-4-hydroxypyrrolidine obtained by the method described in Reference Example while vigorously stirring under an ice water-cooling condition. Thereafter, bis(2-methoxyethyl)aminosulfur trifluoride (Deoxo-Fluor) (1.2 equivalents) was added, followed by stirring at an internal temperature of -5 to 5°C for 1 hour, and the temperature was then raised, followed by stirring at an internal temperature of 10 to 20°C for 7 hours, and then left standing overnight at room temperature (Table 4). The reaction solution was subjected to measurements using HPLC. The results are listed in Table 10.

(Example 33)

This reaction was performed in the same manner as in Example 2 using a fluorinating agent, an additive and a solvent listed in Table 5. However, the solvent was used at 12 multiples. The reaction solution was subjected to measurements using HPLC. The results are listed in Table 11.

(Examples 34 and 35)

This reaction was performed in the same manner as in Example 2 using fluorinating agents, additives and solvents listed in Table 5. However, the solvent was used at 20 multiples. The reaction solution was subjected to measurements using HPLC. The results are listed in Table 11.
[Table 5]

| | Fluorinating agent (Equivalent) | Additive (Equivalent) | Solvent | Multiple of solvent (v/w) |
|---|---|---|---|---|
| Example 33 | Deoxo-Fluor (1.2) | $5HF{\cdot}Et_3N$ (4.0) | Toluene | 12 |
| Example 34 | Deoxo-Fluor (1.2) | $5HF{\cdot}Bt_3N$ (4.0) | Toluene | 20 |
| Example 35 | Deoxo-Fluor (1.4) | $5HF{\cdot}Et_3N$ (4.0) | Toluene | 20 |

(Comparative Examples 1 to 4)

This reaction was performed in the same manner as in Example 1 using fluorinating agents and solvents listed in Table 6. The reaction solution was subjected to measurements using HPLC. The results are listed in Table 7.

(Comparative Examples 5 to 10)

This reaction was performed in the same manner as in Example 2 using fluorinating agents, additives and solvents listed in Table 6. The reaction solution was subjected to measurements using HPLC. The results are listed in Table 7.

[Table 6]

| | Fluorinating agent (Equivalent) | Additive (Equivalent) | Solvent |
|---|---|---|---|
| Comparative Example 1 | $CF_3CHFCF_2NEt_2$ (4.0) | - | $CH_3CN$ |
| Comparative Example 2 | $FCHClCF_2NEt_2$ (4.0) | - | $CH_3CN$ |
| Comparative Example 3 | m-tol$CF_2$NEt2 (4.0) | - | $CH_3CN$ |
| Comparative Example 4 | DFI (4.0) | - | $CH_3CN$ |
| Comparative Example 5 | $CF_3(CF_2)_3SO_2F$ (4.0) | DBU (4.0) | $CH_3CN$ |
| Comparative Example 6 | $CF_3CHFCF_2NEt_2$ (4.0) | $5HF{\cdot}Et_3N$ (2.0) | $CH_3CN$ |
| Comparative Example 7 | $FCHClCF_2NEt_2$ (4.0) | $5HF{\cdot}Et_3N$ (2.0) | $CH_3CN$ |
| Comparative Example 8 | DFI (4.0) | $5HF{\cdot}Et_3N$ (2.0) | $CH_3CN$ |
| Comparative Example 9 | m-tol $CF_2NEt_2$(4.0) | $5HF{\cdot}Et_3N$ (2.0) | $CH_3CN$ |
| Comparative Example 10 | p-Tol$SO_2F$ (1.4) | $5HF{\cdot}Et_3N$ (4.0) | $CH_3CN$ |

(Comparative Example 11)

This reaction was performed in the same manner as in Example 2 using Deoxo-Fluor (1.2 equivalents) as the fluorinating agent, triethylamine trihydrofluoride (4.0 equivalents) as the additive and toluene as the solvent. The reaction solution was subjected to measurements using HPLC. The results are listed in Table 8.

The results of Examples 1 to 35 and Comparative Examples 1 to 11 are listed in Table 7 to Table 11.

In Table 7 to Table 11, Conv. (%) means a reaction conversion rate, and the area percentages (%) of a starting material and a product obtained from the HPLC measurement were calculated from the following equation.

$$\text{Reaction conversion rate (\%)} = \text{(Produced amount of desired product)/(Amount of starting material consumed by reaction)} \times 100\ (\%)$$

In Table 7 to Table 11, HPLC (%) means an area percentage (%) of a desired product on completion of the reaction, and was calculated from the following equation.

$$\text{Area percentage (\%) of desired product} = (\text{Produced amount of desired product})/(\text{Peaks except for solvent peaks in total peaks observed by HPLC}) \times 100$$

The obtained isolation yields are listed in the column of results. In this case, a purification method was performed in the same manner as in Example 36. Further, the conditions of HPLC used are as follows.

(HPLC measurement conditions)

Column: GL Sciences Inc. Inertsil ODS-3, 4.6 mm×150 mm
Pre-column: GL Sciences Inc. Inertsil ODS-3, 4.0 mm×10 mm
Mobile phase: diluted phosphoric acid (1→1000) (solution A), acetonitrile (solution B)
0 to 10 min; Linear gradient of solution A:solution B = from 80:20 to 65:35
10 to 20 min; Linear gradient of solution A:solution B = from 65:35 to 5:95
20 to 30 min; solution A:solution B=5:95
Measurement wavelength: 210 nm
Column temperature: 30°C
Flow rate: 1.0 mL/min
Desired product; around 11 min

Here, the diluted phosphoric acid (1→1000) means that 1 mL of phosphoric acid is dissolved in purified water for HPLC, and adjusted to 1000 mL.
[Table 7]

| | Fluorinating agent (Equivalent) | Additive (Equivalent) | Solvent | Conv. (%) | HPLC (%) | Result |
|---|---|---|---|---|---|---|
| Example 1 | Deoxo-Fluor (4.0) | - | $CH_3CN$ | 71.8 | 48.5 | 44% |
| Example 2 | Deoxo-Fluor (4.0) | $5HF \cdot Et_3N$ (2.0) | $CH_3CN$ | 77.8 | 30.7 | - |
| Example 3 | Deoxo-Fluor (1.2) | $5HF \cdot Et_3N$ (10.0) | Toluene | 81.8 | 71.5 | - |
| Example 4 | DAST (1.2) | $5HF \cdot Et_3N$ (10.0) | Toluene | 98.2 | 87.7 | - |
| Example 5 | DAST(1.2) | $5HF \cdot Et_3N$ (4.0) | Toluene | 72.1 | 64.9 | - |
| Example 6 | Morpho-DAST (1.2) | $5HF \cdot Et_3N$ (4.0) | Toluene | 96.2 | 78.5 | - |
| Example 7 | Deoxo-Fluor (1.2) | $5HF \cdot Et_3N$ (4.0) | Toluene | 95.5 | 55.2 | - |
| Comparative Example 1 | $CF_3CHFCF_2NEt_2$ (4.0) | - | $CH_3CN$ | - | - | Complicated reaction mixture |
| Comparative Example 2 | $FCHClCF_2NEt_2$ (4.0) | - | $CH_3CN$ | - | - | Complicated reaction mixture |
| Comparative Example 3 | m-tol$CF_2NEt_2$ (4.0) | - | $CH_3CN$ | - | - | Complicated reaction mixture |
| Comparative Example 4 | DFI (4.0) | - | $CH_3CN$ | - | - | Complicated reaction m ixture |
| Comparative Example 5 | $CF_3(CF_2)_3SO_2F$ (4.0) | DBU (4.0) | $CH_3CN$ | - | - | Complicated reaction mixture |

(continued)

| | Fluorinating agent (Equivalent) | Additive (Equivalent) | Solvent | Conv. (%) | HPLC (%) | Result |
|---|---|---|---|---|---|---|
| Comparative Example 6 | $CF_3CHFCFNEt_2$ (4.0) | $5HF\cdot Et_3N$ (2.0) | $CH_3CN$ | 28.4 | 15.8 | Complicated reaction mixture |
| Comparative Example 7 | $FCHClCF_2NEt_2$ (4.0) | $5HF\cdot Et_3N$ (2.0) | $CH_3CN$ | - | - | Complicated reaction mixture |
| Comparative Example 8 | DFI (4.0) | $5HF\cdot Et_3N$ (2.0) | $CH_3CN$ | - | - | Complicated reaction mixture |
| Comparative Example 9 | m-tol$CF_2NEt_2$ (4.0) | $5HF\cdot Et_3N$ (2.0) | $CH_3CN$ | - | - | Complicated reaction mixture |
| Comparative Example 10 | p-Tol$SO_2F$ (1.4) | $5HF\cdot Et_3N$ (4.0) | $CH_3CN$ | - | - | Complicated reaction mixture |

As described in Comparative Examples 1 to 10 in Table 7, a variety of fluorinating agents were examined. As a result, it was found that when the fluorinating agent other than the sulfur tetrafluoride derivative was used, the desired product was not obtained, and became a complicated reaction mixture. On the other hand, it was found that the fluorination reaction proceeds when the sulfur tetrafluoride derivative was used, as shown in Examples 1 to 7.

[Table 8]

| | Fluorinating agent (Equivalent) | Additive (equivalent) | Solvent | Conv. (%) | HPLC (%) | Result |
|---|---|---|---|---|---|---|
| Example 1 | Deoxo-Fluor (4.0) | - | $CH_3CN$ | 71.8 | 48.5 | 44% |
| Example 8 | Deoxo-Fluor (4.0) | - | $CH_3CN$ (1,194 ppm, $H_2O$) | 87 | 38.6 | 53% |
| Example 9 | Deoxo-Fluor (4.0) | - | $CH_3CN$ (7,358 ppm, $H_2O$) | 92.2 | - | 62% |
| Example 10 | Deoxo-Fluor (4.0) | $5HF\cdot Et_3N$ (1.0) | $CH_3CN$ | 90 | - | 57% |
| Example 11 | Deoxo-Fluor (1.4) | HF·Pyridine (4.0) | Toluene | 80.7 | 31.4 | - |
| Comparative Example 12 | Deoxo-Fluor (1.2) | $3HF\text{-}Et_3N$ (4.0) | Toluene | 13.1 | 6.7 | - |

As described in Examples 8 to 11 in Table 8, it was found that when water (Examples 8 and 9), or triethylamine pentahydrofluoride (Example 10) or a hydrogen fluoride pyridine complex (Example 11) as the hydrogen fluoride source was added to the reaction system, the fluorination reaction was accelerated, compared to Example 1 in which the water or hydrogen fluoride source was not added.

On the other hand, as described in Comparative Example 12, triethylamine trihydrofluoride did not show an effect of accelerating the fluorination reaction. Unlike triethylamine pentahydrofluoride, triethylamine trihydrofluoride does not contain an excessive amount of hydrogen fluoride with respect to triethylamine, and thus, was considered not to show an effect of accelerating the fluorination reaction.

[Table 9]

| | Fluorinating agent (Equivalent) | Additive (Equivalent) | Solvent | Conv. (%) | HPLC (%) | Result |
|---|---|---|---|---|---|---|
| Example 10 | Deoxo-Fluor (4.0) | $5HF\text{-}Et_3N$ (1.0) | $CH_3CN$ | 90 | - | 57% |
| Example 12 | Deoxo-Fluor (4.0) | $5HF\cdot Et_3N$ (2.0) | $CH_3CN$ | 77.8 | 30.7 | - |
| Example 13 | Deoxo-Fluor (1.5) | $5HF\cdot Et_3N$ (2.0) | $CH_3CN$ | 78.4 | 38.6 | - |
| Example 14 | Deoxo-Fluor (1.2) | $5HF\cdot Et_3N$ (2.0) | $CH_3CN$ | 80.5 | 47.7 | - |
| Example 15 | Deoxo-Fluor (1.0) | $5HF\cdot Et_3N$ (2.0) | $CH_3CN$ | 89.7 | 51.7 | 75% |
| Example 16 | Deoxo-Fluor (1.0) | $5HF\cdot Et_3N$ (2.4) | $CH_3CN$ | 87.7 | 57.7 | - |
| Example 17 | Deoxo-Fluor (1.0) | $5HF\cdot Et_3N$ (3.0) | $CH_3CN$ | 88.1 | 74.8 | - |
| Example 18 | Deoxo-Fluor (1.2) | $5HF\cdot Et_3N$ (4.0) | $CH_3CN$ | 95.7 | 81.2 | - |
| Example 19 | Deoxo-Fluor (1.5) | $5HF\cdot Et_3N$ (4.0) | $CH_3CN$ | 94.9 | 69.8 | - |
| Example 20 | Deoxo-Fluor (1.2) | $5HF\cdot Et_3N$ (3.4) | $CH_3CN$ | 95.3 | 70.9 | - |
| Example 21 | Deoxo-Fluor (1.2) | $5HF\cdot Et_3N$ (10.0) | Toluene | 81.8 | 71.5 | - |

As described in Examples 12 to 21 in Table 9, the equivalence of the triethylamine pentahydrofluoride added as a hydrogen fluoride source was examined. As a result, it was found that the equivalence of the fluorinating agent could be decreased to 1 to 1.5 equivalents by adding 2 equivalents or more of triethylamine pentahydrofluoride.

As described in Examples 17 to 21, it was also found that the HPLC relative purity of a product was improved on completion of the fluorination reaction by using 3 equivalents or more of triethylamine pentahydrofluoride.

[Table 10]

| | Fluorinating agent (Equivalent) | Additive (Equivalent) | Solvent | Conv. (%) | HPLC (%) | Result |
|---|---|---|---|---|---|---|
| Example 1 | Deoxo-Fluor (4.0) | - | $CH_3CN$ | 71.8 | 48.5 | 44% |
| Example 7 | Deoxo-Fluor (1.2) | $5HF\cdot Et_3N$ (4.0) | Toluene | 95.5 | 55.2 | - |
| Example 18 | Deoxo-Fluor (1.2) | $5HF\cdot Et_3N$ (4.0) | $CH_3CN$ | 95.7 | 81.2 | - |
| Example 22 | Deoxo-Fluor(4.0) | - | THF | - | - | 34% |
| Example 23 | Deoxo-Fluor (4.0) | - | DME | - | - | 39% |
| Example 24 | Deoxo-Fluor (4.0) | - | $CH_2Cl_2$ | - | - | 52% |
| Example 25 | Deoxo-Fluor (4.0) | - | Toluene | - | - | 30% |
| Example 26 | Deoxo-Fluor (1.2) | $5HF\cdot Et_3N$ (4.0) | $CHCl_3$ | 90.2 | 80.1 | - |
| Example 27 | Deoxo-Fluor (1.2) | $5HF\cdot Et_3N$ (4.0) | THF | 89.2 | 58.8 | - |
| Example 28 | Deoxo-Fluor (1.5) | $5HF\cdot Et_3N$ (4.0) | $CHCl_3$ | 91 | 79.3 | - |
| Example 29 | Deoxo-Fluor (1.5) | $5HF\cdot Et_3N$ (4.0) | Toluene | 93.5 | 90 | - |
| Example 30 | Deoxo-Fluor (1.4) | $5HF\cdot Et_3N$ (4.0) | Toluene | 97.6 | 85.9 | 86% |
| Example 31 | Deoxo-Fluor (1.4) | $5HF\cdot Et_3N$ (4.0) | AcOEt | 76.9 | 60.5 | - |
| Example 32 | Deoxo-Fluor (1.2) | $5HF\cdot Et_3N$ (4.0) | - (neat) | 95.2 | 73.3 | - |

As described in Examples 1, 7, 18 and 22 to 31 in Table 10, it was found that the fluorination reaction can proceed in a variety of solvents such as acetonitrile, tetrahydrofuran (THF), 1,2-dimethoxyethane (DME), dichloromethane, chloroform, toluene and ethyl acetate.

As described in Example 32, it was found that the fluorination reaction can proceed in the absence of the solvent.

[Table 11]

| | Fluorinating agent (Equivalent) | Additive (Equivalent) | Solvent | Multiple of solvent (v/w) | Conv. (%) | HPLC (%) | Result |
|---|---|---|---|---|---|---|---|
| Example 7 | Deoxo-Fluor (1.2) | $5HF \cdot Et_3N$ (4.0) | Toluene | 6 | 95.5 | 55.2 | - |
| Example 33 | Deoxo-Fluor (1.2) | $5HF \cdot Et_3N$ (4.0) | Toluene | 12 | 93.4 | 84.5 | 81% |
| Example 34 | Deoxo-Fluor (1.2) | $5HF \cdot Et_3N$ (4.0) | Toluene | 20 | 95.8 | 84.9 | 74% |
| Example 35 | Deoxo-Fluor (1.4) | $5HF \cdot Et_3N$ (4.0) | Toluene | 20 | 98 | 82.3 | 84% |

As described in Examples 33 to 35 in Table 11, it was found that the fluorination reaction can proceed even when the solvent was used at 6 multiples or more.

(Example 36)

Benzyl (3S,4S)-3-(cyclopropylaminomethyl)-4-fluoropyrrolidine-1-carboxylate hydrochloride

[Chem. 13]

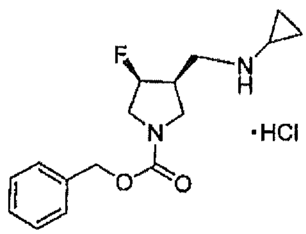

454 g (equivalent to 1.50 mol, including a solvent) of benzyl (3S,4R)-3-(cyclopropylaminomethyl)-4-hydroxypyrrolidine-1-carboxylate was dissolved in 0.98 L of acetonitrile, concentrated under reduced pressure, and dissolved again in 1.47 L of acetonitrile. 1.11 L (6.02 mol) of bis(2-methoxyethyl)aminosulfur trifluoride (Deoxo-Fluor) was added dropwise at an internal temperature of -25 to -5°C, followed by stirring at an internal temperature of -10 to -5°C for 1 hour, and the temperature was raised, followed by stirring at an internal temperature of 10 to 25°C for 2 hours, and then left standing overnight at room temperature.

The reaction solution was added to 12.3 L of ice water, and then adjusted to pH 9.5 by adding a 5 mol/L sodium hydroxide solution at an internal temperature of 0 to 10°C. The mixture was extracted with 12.3 L of ethyl acetate, washed with 4.90 L of saturated saline, and 1.23 kg of anhydrous sodium sulfate was then added, followed by stirring for 0.5 hours. Anhydrous magnesium sulfate was separated by filtration, washed with 0.98 L of ethyl acetate, and the filtrate and the washing were combined, and then concentrated under reduced pressure.

The obtained residue (607 g, HPLC relative purity: 58%) was dissolved in 0.98 L of toluene, charged into 3.43 kg of NH silica gel filled with toluene, and eluted with toluene to collect a fraction containing a desired product, which was then concentrated under reduced pressure. The residue (370 g, HPLC relative purity: 77%) was dissolved in 2.45 L of ethyl acetate, and 1.0 L of hydrogen chloride/methanol was added, followed by stirring at room temperature.

After the crystallization, the mixture was stirred at room temperature for 0.5 hours, and 5.88 L of diisopropyl ether was added, followed by stirring at room temperature for 0.5 hours, and then stirred at an internal temperature of 1 to 10°C for 0.5 hours. The precipitated solid was collected by filtration, and washed with 1.96 L of a mixed solution of a diisopropyl ether:ethyl acetate (3:1) mixture. The solid was dried at 60°C to obtain 275 g of a title compound as a white powder (yield: 56%).

mp (hot plate method); 159-162°C (decomp.)
ESI-MS (positive) m/z: 293 [M+ H-HCl]$^+$
Anal. Calcd for $C_{16}H_{22}ClFN_2O_2$: C, 58.44; H, 6.74; N, 8.52. Found: C, 58.12; H, 6.68; N, 8.43.
$^1$H-NMR(CDCl$_3$, 400MHz)δ: 0.78-0.92(2H, m), 1.16-1.34(2H, m), 2.49-2.61(1H, m), 2.87-3.20(2H, m), 3.27(1H, dt, J=10.8, 23.5Hz), 3.34-3.41(1H, m), 3.63(1H, ddt, J=3.6, 13.5, 40.6Hz), 3.72-3.90(1H, m), 3.96(1H, dd, J=8.8, 10.3Hz), 5.07-5.17(2H, m), 5.48(1H, ddt, J=2.9, 27.6, 52.7), 7.28-7.39(5H, m), 9.86(2H, br s).
HPLC relative purity: 98.4%(RT: 11.39 min) measurement wavelength: 210 nm, column: Inertsil ODS-3, 4.6 mm×150 mm, pre-column: Inertsil ODS-3, 4.0 mm×10 mm, mobile phase: diluted phosphoric acid (1→1000) (solution A), acetonitrile (solution B), 0 to 10 min; linear gradient of solution A:solution B = from 80:20 to 65:35, 10 to 20 min; linear gradient of solution A:solutionB = from 65:35 to 5:95, 20 to 30 min; solution A:solution B=5:95, column temperature: 30°C, flow rate: 1.0 mL/min

(Example 37)

Benzyl (3S,4S)-3-(cyclopropylaminomethyl)-4-fluoropyrrolidine-1-carboxylate hydrochloride

[Chem. 14]

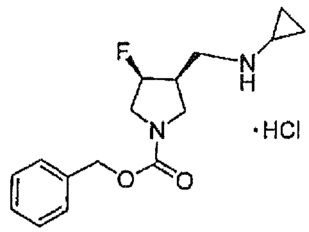

266 g (814 mmol) of benzyl (3S,4R)-3-(cyclopropylaminomethyl)-4-hydroxypyrrolidine-1-carboxylate hydrochloride was dissolved in 0.80 L of water, and 0.61 L of a 2 mol/L sodium hydroxide solution was added at an internal temperature of - 5 to 10°C, followed by stirring and extracting twice with 2.66 L and 1.33 L of toluene. Organic layers were combined, and washed with 0.80 L of a 10% sodium sulfate solution, and 266 g of anhydrous magnesium sulfate was added, followed by stirring for 1 hour. The anhydrous magnesium sulfate was separated by filtration, and the solid was washed with 0.80 L of toluene. The filtrate and the washing were combined, and then concentrated under reduced pressure to obtain 245 g of benzyl (3S,4R)-3-((cyclopropylamino)methyl)-4-hydroxypyrrolidine-1-carboxylate as a colorless oily product.
The obtained compound was dissolved in 3.19 L of toluene, and 655 g (3.26 mol) of triethylamine pentahydrofluoride was added at an internal temperature of -5 to 10°C, followed by stirring at an internal temperature of -5 to 5°C for 0.5 hours. 210 mL (1.14 mol) of bis(2-methoxyethyl)aminosulfur trifluoride (Deoxo-Fluor) was added to the mixture at an internal temperature of -5 to 5°C, followed by stirring at an internal temperature of -5 to 5°C for 1 hour, and the temperature was then raised, followed by stirring at an internal temperature of 10 to 20°C for 7 hours, , and then left standing overnight at room temperature.
The reaction solution was added to 6.65 L of ice water, and then adjusted to pH 2.5 by adding 0.53 L of 6 mol/L hydrochloric acid. The mixture was washed with 2.13 L of toluene, and adjusted to pH 9.8 by adding a 5 mol/L potassium hydroxide solution to the aqueous layer at an internal temperature of 0 to 10°C. The mixture was extracted twice with 7.98 L and 3.99 L of toluene, and the organic layers were combined, and then washed with 2.66 L of saturated saline, and 266 g of anhydrous magnesium sulfate was added, followed by stirring for 0.5 hours. The anhydrous magnesium sulfate was separated by filtration, washed with 1.25 L of toluene, and the filtrate and the washing were combined, and then concentrated under reduced pressure.
The obtained residue (265 g, HPLC relative purity: 91%) was dissolved in 0.53 L of toluene, charged into 798 g of NH silica gel filled with toluene, and eluted with approximately 8 L of toluene, and the eluate was concentrated under reduced pressure. The residue (HPLC relative purity: 95%) was dissolved in 1.33 L of ethyl acetate, 0.71 L of a hydrogen chloride/methanol solution was added at room temperature, followed by heating, and 5.32 L of diisopropyl ether was added at an internal temperature of 35 to 45°C to form crystals, followed by stirring for 15 minutes at a temperature close to the crystallization temperature. 2.66 L of diisopropyl ether was added, stirred for 0.5 hours, cooled, and then stirred at an internal temperature of 1 to 10°C for 0.5 hours. The precipitated solid was collected by filtration, and washed

with 2.66 L of diisopropyl ether. The solid was dried under reduced pressure at 50°C to obtain 214 g of a title compound as a white powder (yield: 80%).

mp (hot plate method); 160-162°C (decomp.)
ESI-MS (positive) m/z: 293 $[M+H-HCl]^+$
Anal. Calcd for $C_{16}H_{22}ClFN_2O_2$: C, 58.44; H, 6.74; N, 8.52. Found: C, 58.18; H, 6.67; N, 8.54.

$^1$H-NMR($CDCl_3$, 400 MHz)δ: 0.78-0.92(2H, m), 1.16-1.34(2H, m), 2.49-2.61(1H, m), 2.87-3.20(2H, m), 3.27 (1H, dt, J-10.8, 23.5Hz), 3.34-3.41(1H, m), 3.63(1H, ddt, J=3.6, 13.5, 40.6Hz), 3.72-3.90(1H, m), 3.96(1H, dd, J=8.8, 10.3Hz), 5.07-5.17(2H, m), 5.48(1H, ddt, J=2.9, 27.6, 52.7), 7.28-7.39(5H, m), 9.86(2H, br s).

HPLC relative purity: 99.3% (RT: 11.11 min) measurement wavelength: 210 nm, column: Inertsil ODS-3, 4.6 mm×150 mm, pre-column: Inertsil ODS-3, 4.0 mm×10 mm, mobile phase: diluted phosphoric acid (1→1000) (solution A), acetonitrile (solution B), 0 to 10 min; linear gradient of solution A:solution B = from 80:20 to 65:35, 10 to 20 min; linear gradient of solution A:solution B = from 65:35 to 5:95, 20 to 30 min; solution A:solution B=5:95, column temperature: 30°C, flow rate: 1.0 mL/min

(Example 38)

(3R,4S)-3-(cyclopropylaminomethyl)-4-fluoropyrrolidine dihydrochloride

[Chem. 15]

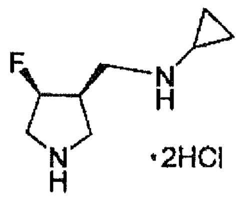

213 g (0.648 mol) of benzyl (3S,4S)-3-(cyclopropylaminomethyl)-4-fluoropyrrolidine-1-carboxylate hydrochloride was added to a mixture solution of 533 mL of purified water and 533 mL of 35% hydrochloric acid, followed by stirring at an internal temperature of 80 to 90°C for 2 hours. The reaction solution was washed twice with 852 mL of ethyl acetate, and 10.7 g of activated carbon was added, followed by stirring for 1.5 hours. The activated carbon was separated by filtration, and washed with 852 mL of purified water. The filtrate and the washing were combined, and concentrated under reduced pressure.

639 mL of ethanol was added to the residue, followed by concentration under reduced pressure, and 639 mL of ethanol was added again, followed by concentration under reduced pressure. 639 mL of ethanol was added to the residue and heated, and 63.9 mL of purified water was added at an internal temperature of 65 to 75°C, followed by dissolving. The residue was cooled, followed by stirring at an internal temperature of 35 to 45°C for 30 minutes, and 639 mL of ethyl acetate was then added, followed by stirring at an internal temperature of 35 to 45°C for 30 minutes. The residue was cooled, followed by stirring at an internal temperature of 20°C or lower for 30 minutes. The precipitated solid was collected by filtration, and washed with 639 mL of an ethyl acetate-ethanol mixture solution (1:1). The solid was dried at 50°C under reduced pressure to obtain 137 g of crude crystals as a white powder (91%).

137 g of the obtained crude crystals was added to 410 mL of ethanol, and heated, and 48 mL of purified water was added, followed by dissolving (internal temperature of 78°C). The crude crystals were cooled, followed by stirring at an internal temperature of 35 to 45°C for 30 minutes, and 410 mL of ethyl acetate was added, followed by stirring at an internal temperature of 35 to 45°C for 30 minutes. The crude crystals were cooled, followed by stirring at an internal temperature of 10°C or lower for 30 minutes. The precipitated crystals were collected by filtration, and washed with 410 mL of an ethyl acetate-ethanol mixture solution (1:1). The crystals were dried at 50°C under reduced pressure to obtain 130 g of a title compound as a white powder (87%).

mp (hot plate method); 206-208°C (decomp.)
$[a]_D^{22}$-19.7 (c 0.3, $H_2O$).
CI-MS (positive) m/z: 159$[M+H]^+$
Anal. Calcd for $C_8H_{17}Cl_2FN_2$: C, 41.57; H, 7.41; N, 12. 12. Found: C, 41.43; H, 7.45; N, 12.08.

$^1$H-NMR(DMSO-$d_6$, 400MHz)δ: 0.69-0.82(2H, m), 0.92-1.05(2H, m), 2.65-2.91(2H, m), 3.05(1H, t, J=11.7Hz), 3.14(1H, dd, J=7.2, 12.8Hz), 3.30(1H, dd, J=6.1, 12.7Hz), 3.43-3.55(2H, m), 3.62(1H, dd, J=8.3, 11.5Hz), 5.40-5.57(1H,

m), 9.86(4H, br s).

GC relative purity: 96.1% (RT: 9.91 min), column: HP-1 Methyl Siloxane, 0.5 mm×30 m, front inlet temperature: 210°C, split ratio: 10:1, initial oven temperature: 100°C, heating rate: 10°C/min, final temperature: 240°C, final time: 6 min, front detector temperature: 250°C, hydrogen flow rate: 40.0 mL/min, air flow rate: 400.0 mL/min, makeup flow rate: 25.0 mL/min.

(Example 39)

(3R,4S)-3-(cyclopropylaminomethyl)-4-fluoropyrrolidine dihydrochloride

[Chem. 16]

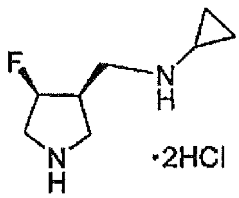

2 mL of hydrochloric acid and 132 mg of a 10% palladium-carbon carrier (containing 50% moisture) were added to a suspension solution of 1.32 g (4.00 mmol) of benzyl (3S,4S)-3-(cyclopropylaminomethyl)-4-hydroxypyrrolidine-1-carboxylate hydrochloride in 9.2 mL of ethanol, followed by stirring at room temperature for 3 hours under a hydrogen atmosphere. The catalyst was separated by filtration, and washed with 9.2 mL of ethanol, and the filtrate and the washing were combined, followed by stirring at room temperature to precipitate crystals. After the crystallization, 18.4 mL of ethyl acetate was added dropwise, followed by stirring at room temperature for 5 hours. The precipitated crystals were collected by filtration, washed with 4.6 mL of ethanol, and then dried at 60°C under reduced pressure to obtain 764 mg of a desired title compound (83%).

mp (hot plate method); 204-206°C (decomp.)

$^1$H-NMR(DMSO-$d_6$, 400MHz)δ: 0.69-0.82(2H, m), 0.92-1.07(2H, m), 2.65-2.91(2H, m), 3.06(1H, t, J=11.7Hz), 3.13(1H, dd, J=7.2, 12.8Hz), 3.30(1H, dd, J=6.1, 12.8Hz), 3.43-3.58(2H, m), 3.62(1H, dd, J=8.3, 11.5Hz), 5.37-5.61(1H, m), 9.80 (4H, br s).

(Example 40)

Benzyl (3R,4R)-3-(cyclopropylaminomethyl)-4-fluoropyrrolidine-1-carboxylate hydrochloride

[Chem. 17]

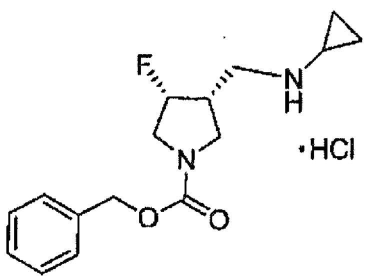

40.0 g (122 mmol) of benzyl (3R,4S)-3-(cyclopropylaminomethyl)-4-hydroxypyrrolidine-1-carboxylate hydrochloride was dissolved in 120 mL of ice water, 20.0 g of sodium chloride was added, and a sodium hydroxide solution (using a total amount obtained by dissolving 7.34 g of sodium hydroxide in 80 mL of purified water) was added followed

by stirring under an ice water-cooling condition.

The mixture was extracted with 200 mL of ethyl acetate, the organic layer was sequentially washed with 200 mL of 10% saline and 200 mL of saturated saline, and 30.0 g of anhydrous sodium sulfate was added, followed by stirring for 1.5 hours. The anhydrous sodium sulfate was separated by filtration, the solid was washed with 0.49 L of ethyl acetate, and the filtrate and the washing were then combined, and concentrated under reduced pressure to obtain 36.8 g of benzyl (3R,4S)-3-((cyclopropylamino)methyl)-4-hydroxypyrrolidine-1-carboxylate as a colorless oily product.

The oily product was dissolved in 80.0 mL of acetonitrile, concentrated under reduced pressure, and then dissolved in 120 mL of dehydrated acetonitrile. The mixture was cooled, 87 mL (492 mmol) of bis(2-methoxyethyl) aminosulfur trifluoride (Deoxo-Fluor) was added dropwise at an internal temperature of -27 to -4°C, followed by stirring at an internal temperature of -15 to -5°C for 1 hour, and the temperature was slowly raised, followed by stirring at an internal temperature of 20 to 25°C for 2 days. The reaction solution was added to 1.0 L of ice water, and adjusted to pH 9.5 by adding 423 g of a 5 mol/L sodium hydroxide solution at an internal temperature of 18°C or lower.

The mixture was extracted with 1.0 L of ethyl acetate, and washed with 500 mL of saturated saline, and 100 g of anhydrous sodium sulfate and 10.0 g of activated carbon were added, followed by stirring at room temperature for 2 hours. The solid was separated by filtration, and washed with 120 mL of ethyl acetate, and the filtrate and the washing were then combined into one, and concentrated under reduced pressure. The obtained residue (47.9 g) was dissolved in toluene, charged into 280 g of a silica gel column (produced by FUJI SILYSIA CHEMICAL LTD., NH) filled with toluene, and eluted with toluene to collect a fraction containing a desired product, which was then concentrated under reduced pressure.

The residue (25.6 g) was dissolved in 200 mL of ethyl acetate, and 89 mL of a 5% hydrogen chloride/methanol solution and 320 mL of diisopropyl ether were added, followed by stirring at room temperature. After the crystallization, the mixture was stirred at room temperature for 0.5 hours, and 480 mL of diisopropyl ether was added, stirred at room temperature for 0.5 hours, and then stirred at an internal temperature of 1 to 10°C for 0.5 hours. The precipitated crystals were collected by filtration, and washed with 160 mL of a diisopropyl ether: ethyl acetate (3:1) mixture solution. The crystals were dried at 60°C to obtain 21.4 g of a partially purified title compound.

214 mL of ethanol was added to 21.4 g of the partially purified product, followed by heating and dissolving. 320 mL of diisopropyl ether was added to the mixture, crystallized, and then stirred for 0.25 hours around an internal temperature of 50°C. 107 mL of diisopropyl ether was added to the mixture, stirred for 0.5 hours around an internal temperature of 50°C, cooled, and then stirred at an internal temperature of 2 to 10°C for 0.5 hours. The precipitated crystals were collected by filtration, and washed with 85 mL of a diisopropyl ether:ethyl acetate (9:1) mixture solution. The wet crystals were dried at 50°C to obtain 17.7 g of a title compound as light reddish yellow powder (50%).

mp (hot plate method); 161-163°C (decomp.)

ESI-MS (positive) m/z: 293[M+H-HCl]$^+$

$^1$H-NMR($CDCl_3$, 400MHz)$\delta$: 0.65-0.82(2H, m), 0.84-1.02(2H, m), 2.63-2.92(2H, m), 3.05-3.22(2H, m), 3.28(1H, dd, J=6.1, 12.5Hz), 3.50-3.75(2H, m), 3.79(1H, dd, J=9.2, 19.1Hz), 5.05-5.05(2H, m), 5.36(1H, d, J=54.8), 7.28-7.42 (5H, m), 9.32(1H, br s), 9.49(1H, br s).

HPLC relative purity: 98.7% (RT: 16.2 min) measurement wavelength: 210 nm, column: Inertsil ODS-3, 4.6 mm×150 mm, pre-column: Inertsil ODS-3, 4.0 mm×10 mm, mobile phase: diluted phosphoric acid (→1000) (solution A), acetonitrile (solution B), 0 min to 10 min; solution A: solution B=85:15, 10 to 40 min; linear gradient of solution A: solution B = from 85:15 to 10:90, column temperature: 30°C, flow rate: 1.0 mL/min

(Example 41)

(3S,4R)-3-(cyclopropylaminomethyl)-4-fluoropyrrolidine dihydrochloride

[Chem. 18]

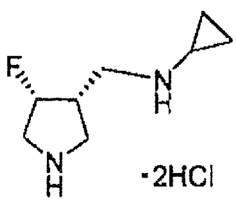

13.0 g (39.5 mmol) of benzyl (3R,4R)-3-(cyclopropylaminomethyl)-4-hydroxypyrrolidine-1-carboxylate was added to a mixture solution of 32.5 mL of purified water and 32.5 mL of 35% hydrochloric acid, followed by stirring at an internal temperature of 75 to 85°C for 2 hours. The reaction solution was cooled, and then washed with 65 mL of ethyl acetate, and 0.65 g of activated carbon was added, followed by stirring at room temperature for 0.5 hours. The activated carbon was separated by filtration, and washed with 52 mL of purified water. The filtrate and the washing were combined, and concentrated under reduced pressure.

39 mL of ethanol was added to the residue, followed by concentration under reduced pressure, and 39 mL of ethanol was added again, followed by concentration under reduced pressure. 39 mL of diisopropyl ether and 26 mL of ethanol were added dropwise to the residue, followed by stirring at an internal temperature of 55°C for 0.5 hours. The residue was cooled, followed by stirring at an internal temperature of 20°C or lower for 1.5 hours. The precipitated crystals were collected by filtration, and washed with 39 mL of a diisopropyl ether-ethanol mixture solution (2:1). The wet crystals were dried at 50°C to obtain 8.40 g of crude crystals as a light brown prism.

42 mL of ethanol was added to 8.40 g of the obtained crude crystals and heated, and the crude crystals dissolved around an internal temperature of 70°C by adding 4.2 mL of purified water. After the cooling and crystallization (the crystallization starts at an internal temperature of 43°C), the mixture was stirred for 10 minutes at a temperature close to the crystallization temperature, and then stirred at an internal temperature of 38 8 to 45°C for 30 minutes, and 42 mL of ethyl acetate was added, followed by stirring at an internal temperature of 45 to 50°C for 0.5 hours. The crystals were cooled, followed by stirring at an internal temperature of 20°C or lower for 0.5 hours. The precipitated crystals were collected by filtration, and washed with 26 mL of an ethyl acetate-ethanol mixture solution (1:1). The wet crystals were dried at 50°C to obtain 7.85 g of a title compound as a light brown prism (86%).

mp(hot plate method); 206-207°C(decomp.)

$[\alpha]_D^{23}$+ 21.7(c 0.3, $H_2O$).

$^1$H-NMR(DMSO-$d_6$, 400MHz)$\delta$: 0.68-0.82(2H, m), 0.92-1.06(2H, m), 2.65-2.91(2H, m), 3.06(1H, t, J=11.7Hz), 3.13(1H, dd, J=7.2, 12.8Hz), 3.30(1H, dd, J=6.1, 12.7Hz), 3.42-3.59(2H, m), 3.62(1H, dd, J=8.2, 11.4Hz), 5.49(1H, dt, J=2.9, 53.6Hz), 9.81(4H, br s).

GC relative purity: 96.4% (RT: 10.09 min), column: HP-1 Methyl Siloxane, 0.53 mmx 30 m, front inlet temperature: 210°C, split ratio: 10:1, initial oven temperature: 100°C, heating rate: 10°C/min, final temperature: 240°C, final time: 6 min, front detector temperature: 250°C, hydrogen flow rate: 40.0 mL/min, air flow rate: 400.0 mL/min, makeup flow rate: 25.0 mL/min.

Although the present invention has been described in detail with reference to certain embodiments, it is apparent to those skilled in the art that various modifications and changes may be made without departing from the spirit and scope of the present invention. Meanwhile, this application is based on Japanese patent application (Patent Application No. 2010-002665) filed on January 8, 2010, the disclosure of which is incorporated herein by reference in its entirety.

## Industrial Applicability

The present invention is an inexpensive and industrially advantageous method for preparing (3R,4S)-3-(cyclopropyl aminomethyl)-4-fluoropyrrolidine or it's enantiomer, or their salts which can be an intermediate for producing pharmaceuticals. The present invention is useful in that it can produce in an industrially advantageous manner a derivative of 7-(3-(cyclopropyl aminomethyl)-4-fluoropyrrolidinyl)quinolonecarboxylic acid, which is safe, shows potent antibacterial activity and also has an effect on resistant bacteria on which conventional antibacterial agents hardly show their effects.

## Claims

**1.** A method for preparing a compound represented by the following general formula (2) or it's enantiomer, or their salts, comprising:

a fluorination step of fluorinating a 4-hydroxy-3-(N-substituted aminomethyl)pyrrolidine derivative represented by the following general formula (1) or an enantiomer thereof using a sulfur tetrafluoride derivative

[Chem. 1]

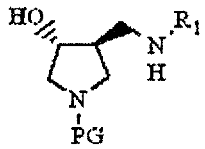

(1)

[In the formula (1), PG represents a protective group for the amino group, and $R_1$ represents a C1 to C6 alkyl group which may be substituted, or a C3 to C8 cycloalkyl group which may be substituted.]

[Chem. 2]

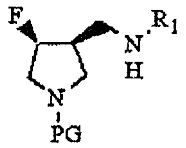

(2)

[In the formula (2), PG represents a protective group for the amino group, and $R_1$ represents a C1 to C6 alkyl group which may be substituted, or a C3 to C8 cycloalkyl group which may be substituted.].

**2.** The production method according to claim 1, further comprising, after the fluorination step:

adding an acid to obtain a salt of a compound represented by the general formula (2) or a salt of an enantiomer thereof, and
further purifying the salt,
wherein the salt of the compound represented by the general formula (2) or the salt of the enantiomer is produced at an HPLC relative purity of 98% or more.

**3.** A method for preparing a compound represented by the following general formula (3) or it's enantiomer, or their salts, comprising step 1 and step 2:

(Step 1) a fluorination step of obtaining a compound represented by the following general formula (2) or it's enantiomer, or their salts thereof by fluorinating the derivative of 4-hydroxy-3-(N-substituted aminomethyl)pyrrolidine represented by the following general formula (1) or an enantiomer thereof using a sulfur tetrafluoride derivative; and
(Step 2) a deprotection step of obtaining a compound represented by the following general formula (3) or it's enantiomer, or their salts by deprotecting the protective group on the amino group of the compound represented by the following general formula (2) or the enantiomer thereof, or a salt thereof obtained in step 1.

[Chem. 3]

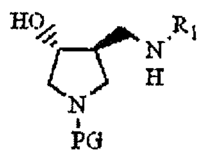

(1)

[In the formula (1), PG represents a protective group for the amino group, and $R_1$ represents a C1 to C6 alkyl group which may be substituted, or a C3 to C8 cycloalkyl group which may be substituted.]

[Chem. 4]

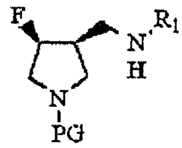
(2)

[In the formula (2), PG represents a protective group for the amino group, and $R_1$ represents a C1 to C6 alkyl group which may be substituted, or a C3 to C8 cycloalkyl group which may be substituted.]

[Chem. 5]

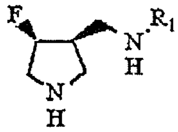
(3)

[In the formula (3), $R_1$ represents a C1 to C6 alkyl group which may be substituted, or a C3 to C8 cycloalkyl group which may be substituted.]

**4.** The production method according to any one of claims 1 to 3, wherein water or a hydrogen fluoride source is added in the fluorination step.

**5.** The production method according to any one of claims 1 to 4, wherein a sulfur tetrafluoride derivative is added in the fluorination step after the addition of the water or hydrogen fluoride source.

**6.** The production method according to claim 4 or 5, wherein the hydrogen fluoride source is a hydrogen fluoride pyridine complex or a triethylamine pentahydrofluoride.

**7.** The production method according to any one of claims 4 to 6, wherein the hydrogen fluoride source is the triethylamine pentahydrofluoride.

**8.** The production method according to any one of claims 1 to 7, wherein the hydrogen fluoride source is used at 3 equivalents or more with respect to the 4-hydroxy-3-(N-substituted aminomethyl)pyrrolidine derivative represented by the general formula (1) or the enantiomer thereof in the fluorination step.

**9.** The production method according to any one of claims 1 to 8, wherein the hydrogen fluoride source is used at 4 equivalents or more with respect to the 4-hydroxy-3-(N-substituted aminomethyl)pyrrolidine derivative represented by the general formula (1) or the enantiomer thereof in the fluorination step.

**10.** The production method according to any one of claims 1 to 9, wherein the sulfur tetrafluoride derivative is bis(2-methoxyethyl)aminosulfur trifluoride (Deoxo-Fluor).

**11.** The production method according to any one of claims 1 to 10, wherein the protective group for the amino group represented by PG is an alkoxycarbonyl group or an aralkoxycarbonyl group.

**12.** The production method according to claim 11, wherein the protective group for the amino group represented by PG is a benzyloxycarbonyl group.

**13.** The production method according to any one of claims 1 to 12, wherein $R_1$ is a cyclopropyl group.

**14.** The production method according to any one of claims 1 to 10, wherein the protective group for the amino group represented by PG is a benzyloxycarbonyl group, and $R_1$ is a cyclopropyl group.

15. A hydrochloride of a compound represented by the general formula (2) or a hydrochloride of an enantiomer thereof

[Chem. 6]

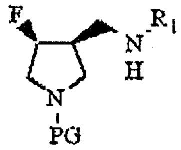

(2)

[In the formula (2), PG represents a protective group for the amino group, and $R_1$ represents a C1 to C6 alkyl group which may be substituted, or a C3 to C8 cycloalkyl group which may be substituted.].

16. A method for purifying a hydrochloride of a compound represented by the general formula (2) or a hydrochloride of an enantiomer thereof to increase the purity

[Chem. 7]

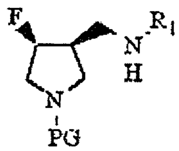

(2)

[In the formula (2), PG represents a protective group for an amino group, and $R_1$ represents a C1 to C6 alkyl group which may be substituted, or a C3 to C8 cycloalkyl group which may be substituted.].

## INTERNATIONAL SEARCH REPORT

International application No.
PCT/JP2011/050206

A. CLASSIFICATION OF SUBJECT MATTER
*C07D207/10*(2006.01)i

According to International Patent Classification (IPC) or to both national classification and IPC

B. FIELDS SEARCHED

Minimum documentation searched (classification system followed by classification symbols)
C07D207/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), CASREACT(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 2008-502689 A (Glaxo Group Ltd.),<br>31 January 2008 (31.01.2008),<br>example 68<br>& WO 2006/02047 A2 & EP 1796466 A2<br>& US 2007/287701 A1 | 1-16 |
| Y | WO 2007/102567 A1 (Kyorin Pharmaceutical Co., Ltd.),<br>13 September 2007 (13.09.2007),<br>examples 7, 9, 18<br>& CA 2645191 A & EP 1992613 A1<br>& US 2009/023935 A1 | 1-16 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 April, 2011 (12.04.11) | 26 April, 2011 (26.04.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/050206

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2002/083636 A1 (Eisai Co., Ltd.), 24 October 2002 (24.10.2002), step C-4 & AU 2002243015 A & EP 1375479 A1 & US 2004/073045 A1 | 1-14 |
| Y | J.Org.Chem., (1960), vol.82, p.543-551, particularly, p.549, Table IX | 1-14 |
| P,Y | J.Org.Cehm., (2010), 75(10), p.3401-11, particularly, ABSTRACT, TABLE2 | 1-14 |
| X<br>Y | JP 61-282382 A (Dainippon Pharmaceutical Co., Ltd.), 12 December 1986 (12.12.1986), referential examples; example 2 (Family: none) | 15,16<br>1-16 |
| X<br>Y | JP 61-189281 A (Dainippon Pharmaceutical Co., Ltd.), 22 August 1986 (22.08.1986), examples; referential examples & EP 191451 A1 & AU 8653216 A & FI 8600556 A & US 4738968 A & ZA 8601074 A & DK 8600717 A & HU 41784 A & SU 1456015 A | 15,16<br>1-16 |
| X<br>Y | WO 03/078439 A1 (Kyorin Pharmaceutical Co., Ltd.), 25 September 2003 (25.09.2003), example 23; referential examples 11 to 13 & CA 2478763 A & AU 2003213335 A & EP 1486500 A1 & BR 2003008643 A & CN 1653071 A & NZ 535182 A & NO 2004003850 A & US 2005/182052 A1 | 15,16<br>1-16 |
| X | JP 2007-511597 A (Warner-Lambert Company L.L.C.), 10 May 2007 (10.05.2007), example 26 & WO 2005/049605 A1 & EP 2546339 A & EP 1687296 A1 & BR 2004016708 A & US 2007/191333 A1 | 15,16 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0307843 A
- WO 05026147 A
- JP 2005239617 A
- WO 07102567 A
- WO 05075426 A
- JP 2008222578 A
- WO 0613048 A
- WO 2003078439 A
- WO 2007102567 A
- WO 200613048 A
- JP 2010002665 A


### Non-patent literature cited in the description


- *Journal of Labelled Compounds & Radiopharmaceuticals,* 2000, vol. 43 (10), 1047-1058
- *Tetrahedron Letters,* 1996, vol. 37 (43), 7743-7744
- *J. Chem. Res.,* 1983, vol. 246
- *Journal of Organic Chemistry,* 1979, vol. 44 (5), 771-777
- *Journal of Organic Chemistry,* 1975, vol. 44 (25), 3808-3809
- **GREEN, T. W. ; WUTS, P. G. M.** Protective Groups in Organic Synthesis. Wiley Interscience Publication, John-Weiley & Sons, 1991, 309-348